# FASCICULE DE BREVET EUROPEEN

(11) **EP 2 919 797 B1**
(45) Date de publication et mention de la délivrance du brevet: **03.05.2017**
(21) Numéro de dépôt: 13792362.9
(22) Date de dépôt: 15.11.2013
(51) Int. Cl.: A61K 36/02, A61K 31/737, A61P 31/10, A61P 33/00, A61K 35/748, A61K 36/03, A61K 36/04, A61K 36/05, A23L 33/105, A23K 20/147, A23K 20/163

(54) **COMPOSITION DE POLYSACCHARIDE SULFATÉ**
SULFATIERTE POLYSACCHARIDZUSAMMENSETZUNG
SULPHATED POLYSACCHARIDE COMPOSITION

(30) Priorité: 16.11.2012 FR 1260941
(43) Date de publication de la demande: 23.09.2015
(73) Titulaire: Université Blaise Pascal Clermont II, 63006 Clermont Ferrand Cedex 1 (FR); Centre National de la Recherche Scientifique (C.N.R.S.), 75016 Paris (FR)
(72) Inventeur: MICHAUD, Philippe, F-63160 Billom (FR); LAROCHE, Céline, F-63350 Crevant-Laveine (FR); VILLAY, Aurore, F-63570 Auzat La Combelle (FR); ROUSSEL, Michaël, F-63000 Clermont Ferrand (FR); DIOGON, Marie, F-63000 Clermont Ferrand (FR); EL ALAOUI, Hicham, F-63100 Clermont Ferrand (FR); DELBAC, Frédéric, F-63170 Aubiere (FR)
(74) Mandataire: Lavoix
(86) Numéro de dépôt international: PCT/EP2013/073995
(87) Numéro de publication internationale: WO 2014/076261

(56) Documents cités:
- WO-A2-02/02189
- JP-A- H1 180 003
- US-A1- 2003 181 416
- JUN-HU CHEN ET AL: "Growth-inhibitory effect of a fucoidan from brown seaweed Undaria pinnatifida on Plasmodium parasites", PARASITOLOGY RESEARCH ; FOUNDED AS ZEITSCHRIFT FÜR PARASITENKUNDE, SPRINGER, BERLIN, DE, vol. 104, no. 2, 13 septembre 2008 (2008-09-13), pages 245-250, XP019716049, ISSN: 1432-1955

## Description

L'invention concerne une composition à base d'au moins un polysaccharide sulfaté, notamment issu d'une algue, et associé à au moins un ingrédient alimentaire pour une utilisation dans le traitement ou la prévention d'une infection provoquée par une microsporidie chez l'homme ou l'animal.
Elle possède des propriétés utiles comme agent antiparasitaire chez l'homme ou l'animal ; pour le traitement ou la prévention d'une infection provoquée par au moins une microsporidie chez l'homme ou l'animal ; en particulier pour le traitement ou la prévention chez l'abeille d'une infection provoquée par la microsporidie *Nosema,* de préférence *Nosema ceranae* ou *Nosema apis,* ou encore pour stimuler les défenses immunitaires de l'abeille.

L'invention concerne également une composition comprenant un polysaccharide sulfaté pour une utilisation comme agent antiparasitaire chez l'homme ou l'animal dans le traitement ou la prévention d'au moins une infection causée par au moins une microsporidie chez l'homme ou l'animal, de préférence par la microsporidie *Nosema* chez l'abeille. L'invention concerne également une composition comprenant un polysaccharide sulfaté pour une utilisation dans méthode pour traiter ou prévenir une infection causée par au moins un parasite chez l'abeille. La divulgation concerne également une méthode de production de miel en présence d'une infection causée par au moins un parasite, comprenant l'administration chez l'abeille d'une composition selon l'invention. La divulgation concerne également une méthode pour maintenir la pollinisation des plantes par des abeilles infectées par au moins un parasite, comprenant l'administration d'une composition selon l'invention. L'invention concerne également une composition pour l'utilisation dans une méthode pour stimuler les défenses immunitaires chez l'abeille, comprenant l'administration d'une composition selon l'invention. US 2003/0181416 concerne une méthode de traitement d'infection microbienne des mammifères au moyen d'un polysaccharide sulfaté.

JP 11080003 divulgue un agent de traitement antiparasitaire comprenant un polysaccharide sulfaté. JP 11080003 divulgue de manière générique fucoidan, rhamnan sulfaté et carraghenane comme polysaccharides.

Chen *et al.* (Growth-inhibitory effect of a fucoidan from brown seaweed Undaria pinnatifida on Plasmodium parasites de Chen et al., publié en septembre 2008) rapporte les résultats d'une étude de l'effet antiparasitaire sur *Plasmodium falciparum* et *Plasmodium berghei* de trois fractions de fucoidane issues d'une algue brune, *Undaria pinnatifida.*

WO 02/02189 divulgue l'utilisation de polysaccharides sulfatés, notamment de la cellulose sulfatée ou du dextran sulfaté, dans des traitements antiparasitaires. Ces polysaccharides peuvent être administrés sous forme de capsules comestibles ou encore sous forme de crème ou de gel.

US 2011/0172156 concerne l'administration de polysaccharides sulfatés pour améliorer la coagulation sanguine chez un patient. Ces polysaccharides peuvent être extraits d'algues brunes.

WO 2009/144711 divulgue une composition nutritive ou cosmétique comprenant un métal et un polysaccharide sulfaté issu d'une microalgue rouge. Cette composition a pour but de permettre l'administration du métal qu'elle contient. Elle est utile pour des traitements nutritionnels ou dermatologiques et possède des propriétés antimicrobiennes.

PT 103983 divulgue l'utilisation de polysaccharides sulfatés en tant qu'agents antiviraux ou antibactériens.

Aucun de ces documents ne concerne la prévention ou le traitement d'une infection provoquée par une microsporidie chez l'homme ou l'animal.

Les microsporidies, organismes eucaryotes unicellulaires apparentés aux champignons, sont des parasites intracellulaires obligatoires formant des spores de petite taille qui peuvent persister dans l'environnement pendant de très longs mois. Parmi les 1 300 espèces actuellement répertoriées, la majorité parasite les arthropodes et les poissons. Certaines microsporidies sont ainsi à l'origine de pertes économiques conséquentes en aquaculture, notamment dans les élevages de saumons ou de crevettes. Plusieurs espèces sont également responsables de diverses infections chez les mammifères y compris chez l'homme.

La nosémose, dont l'agent causal est la microsporidie *Nosema* sp., est l'une des maladies les plus fréquemment rencontrées chez les abeilles adultes. Cette microsporidie s'attaque aux cellules de l'épithélium intestinal de l'insecte et provoque une nosémose aiguë caractérisée par des traces de diarrhée dans les ruches. Elle peut réduire l'espérance de vie des colonies. Récemment, *Nosema ceranae* une microsporidie parasite de l'abeille asiatique (*Apis cerana*), a été détectée dans des colonies d'abeilles européennes (*Apis mellifera*) présentant des signes d'affaiblissement (Higes et al., Nosema ceranae, a new microsporidian parasite in honeybees in Europe. J Invertebr Pathol (2006), 92(2), 93-95 ; Cox-Foster et al., al., A metagenomic survey of microbes in honey bee colony collapse disorder. Science (2007) 318(5848), 283-287).

Étant donnée l'importance écologique (biodiversité), agronomique (rendement agricole) et économique (apiculture) que revêt l'abeille de par ses activités de pollinisation et de production de miel, l'émergence de ce microorganisme a soulevé de nombreuses inquiétudes au sein des communautés scientifique et apicole.

Par ailleurs, l'infection par *Nosema ceranae* provoque une baisse de l'immunité chez l'abeille (Antùnez et al., Immune suppression in the honey bee (Apis mellifera) following infection by Nosema ceranae (Microsporidia). Environ Microbiol (2009) 11 (9), 2284-2290), la rendant ainsi plus susceptible aux autres infections.

La principale molécule utilisée en traitement contre la nosémose est la fumagilline. Toutefois, cette molécule a été récemment interdite dans de nombreux pays européens, dont la France.

Ainsi, la recherche de nouvelles molécules s'avère nécessaire afin d'identifier des traitements antiparasitaires efficaces, sans effet néfaste sur l'hôte parasité et ne générant pas de résidus dangereux dans les denrées alimentaires issues de la ruche.

La composition utilisée selon l'invention permet d'apporter une solution à tout ou partie de ces problèmes ou inconvénients de l'état de la technique.

Ainsi, l'invention fournit une composition comprenant au moins un polysaccharide sulfaté et au moins un ingrédient alimentaire, pour son utilisation dans le traitement ou la prévention d'une infection provoquée par une microsporidie chez l'homme ou l'animal.

Ainsi, l'ingrédient alimentaire de la composition selon l'invention correspond à la portion de cette composition excluant le polysaccharide sulfaté.

De manière avantageuse, la concentration en poids de polysaccharide sulfaté dans la composition selon l'invention va de 0,01 à 2%, préférentiellement de 0,1 à 1%.

De manière préférée, la composition selon l'invention comprend un polysaccharide sulfaté qui est d'origine naturelle.

De manière avantageuse, la composition selon l'invention peut comprendre au moins un polysaccharide sulfaté issu d'une macroalgue, d'une microalgue ou d'une cyanobactérie ou encore un mélange de polysaccharides sulfatés issus d'une macroalgue, d'une microalgue ou d'une cyanobactérie.

Comme exemples de polysaccharides sulfatés d'origine naturelle, la composition selon l'invention comprend préférentiellement au moins un polysaccharide sulfaté issu d'une macroalgue, de préférence choisie parmi les macroalgues rouges, les macroalgues vertes ou les macroalgues brunes.

De manière toute préférée, la composition selon l'invention comprend au moins un polysaccharide sulfaté issu
- d'une macroalgue rouge, notamment choisie parmi les Rhodophycées productrices d'agar, de carraghénanes, de porphyranes, de funoranes ou de galactanes sulfatés complexes, préférentiellement des genres *Gracilaria, Halymenia, Gelidium, Pterocladia, Acanthopeltis, Campylaephora, Ceranium, Euchema , Chondrus, Porphyra, Laurencia, Furcellaria, Gloiopeltis* et *Iridea* ;
- d'une macroalgue verte, notamment choisie parmi les Chlorophycées produisant des polysaccharides de type ulvane, de préférence du genre *Ulva* ;
- d'une macroalgue brune, notamment choisie parmi les Phéophycées productrices de fucanes sulfatés, préférentiellement des genres *Fucus, Aschophyllum* ou *Cladosiphon.*

Les polysaccharides issus des macroalgues rouges sont préférentiellement construits sur la base d'un enchaînement linéaire d'unités 3-β-galactopyranoses et 4-α-galactopyranoses alternant régulièrement. L'unité β-galactose appartient toujours à la série D, alors que l'unité α-galactose est de configuration D chez les carraghénanes et L chez les agarocolloïdes (par exemple l'agar, le porphyrane, le funorane). Par ailleurs, une partie des résidus 4-α-galactopyranoses peut exister sous la forme de 3,6-anhydrogalactose. La forme 3,6-anhydrogalactose est obtenue par une élimination de l'ester sulfate porté par le carbone 6 de l'unité α-galactose liée en 4, sous l'action de galactose-6-sulfurylases lors de la biosynthèse ou par un traitement alcalin. Certains groupements hydroxyles de ces unités galactopyranoses peuvent être sulfatés, méthylés, pyruvilés ou encore substitués par un monosaccharide

Les galactanes sulfatés issus de macroalgues rouges peuvent être représentés par la formule (I) (Delattre et al, Galactans: An Overview of their Most Important Sourcing and Applications as Natural Polysaccharides. Brazilian Archives of Biology and Technology, 2011, 54 : 1075-1092) : 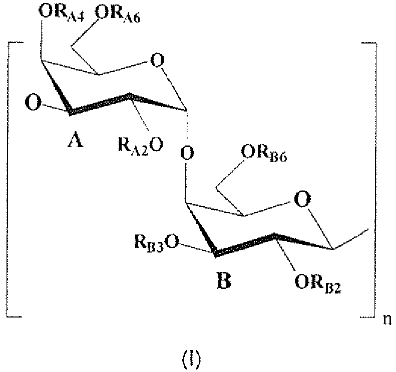 dans laquelle :
- R_{A2} représente un atome d'hydrogène ou SO₃⁻;
- R_{A4} représente un atome d'hydrogène, SO₃⁻ ou l'acide pyruvique;
- R_{A6} représente un atome d'hydrogène, un groupement méthyle, SO₃⁻ ou l'acide pyruvique;
- R_{B2} représente un atome d'hydrogène, un groupement méthyle ou SO₃⁻;
- R_{B3} représente un atome d'hydrogène;
- R_{B6} représente un atome d'hydrogène ou SO₃⁻.

Comme exemples de macroalgues vertes, on peut citer les espèces appartenant aux genres *Ulvella, Ulva, Caulerpa, Codium, Bryopsis.*

En général, trois familles de polysaccharides composent les polysaccharides matriciels d'algues vertes.

La première, majoritaire, est constituée d'un xylorhamnoglycuronane sulfaté désigné par le terme ulvane.

Les polymères de ce type sont solubles dans l'eau et se distinguent des autres polysaccharides extraits d'algues par leur teneur importante en rhamnose (30 à 50 %), en acide glucuronique (10 à 20 %) et en sulfate (16 à 19 %) (Lahaye et al, Structure and function properties of ulvan, a polysaccharide from green seaweeds; Biomacromoleculs 8 (2007) : 1765-1774 ; Lahaye, NMR spectroscopic characterization of oligosaccharides from two Ulva rigida ulvan samples (Ulvales, Chlorophyta) degraded by a lyase. Carbohydrate Research (2008) 314 : 1-12). Chez l'espèce *Ulva lactuca,* les principaux motifs sont composés d'ulvanobiuronate-3-sulfate de type A comprenant du α-L-rhamnose-3-sulfate lié à de l'acide β-D-glucuronique par une liaison de type (1,4) et de l'ulvanobiuronate-3-sulfate de type B comprenant du α-L-rhamnose-3-sulfate lié à de l'acide α-L-iduronique par une liaison de type (1,4) (McKinnel et al, The acid polysaccharide from the green seaweed, Ulva lactuca. Journal of Chemical Society (1962) 398-399.; Quemener et al, Sugar determination in ulvans by a chemical-enzymatic method coupled to high performance anion exchange chromatography. Journal of Applied Phycology (1997) 9 : 179-188). Ces motifs issus de l'ulvane ont été récemment complétés par l'identification de structures de type ulvanobioses (Robic, A., Rondeau-Mouro, C., Sassi, J.F., Lerat, Y., Lahaye M. (2009). Structure and interactions of ulvan in the cell wall of the marine green algae Ulva rotundata (Ulvales, Chlorophyceae). Carbohyd. Pol. 10 : 210-216).

Les ulvanes peuvent être représentés par les formules A₃ₛ, B₃ₛ, U₃ₛ et U_{2'3s} :

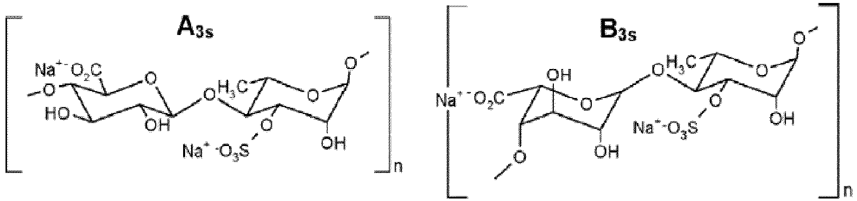

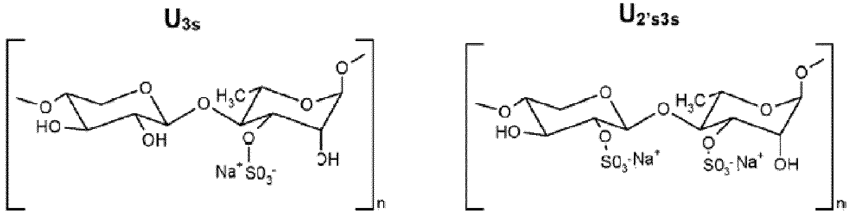

La deuxième famille de polysaccharides est composée de D-xyloglucanes liés en β-(1,4) et de D-glucuronanes liés en β-(1,4). Ces composés peuvent également être représentés par les formules A₃ₛ, B₃ₛ, U₃ₛ et U_{2'3s}.

La dernière famille est représentée par de la «cellulose» amorphe, contenant des résidus xyloses. Chez les algues vertes de l'ordre des Cladophorales et des Codiales, on peut trouver également des xyloarabinogalactanes sulfatés. Ce sont des polymères composés de galactose, d'arabinose et de xylose. Ils comportent environ 17 % de sulfate. Il n'y a pas vraiment d'unité répétitive, mais plutôt des portions comportant des blocs de (1,4)-L-arabinoses, séparés par des unités D-galactoses. Toutes les unités D-xyloses et une partie des unités D-galactoses sont en position terminale.

De manière avantageuse, la macroalgue brune est choisie parmi les Phéophycées productrices de fucanes sulfatés, préférentiellement du genre *Fucus, Aschophyllum* ou *Cladosiphon.*

Les fucanes représentent un ensemble de polysaccharides sulfatés contenant majoritairement du L-fucose mais également d'autres oses comme le galactose, le mannose, le xylose et des acides uroniques (Melo et al, Isolation and characterization of soluble sulphated polysaccharide from the red seaweed Gracilaria cornea. Carbohydrate Polymers (2002) 49 : 491-498) dont la teneur est inférieure à 10 % (Berteau et al, Sulfated fucans, fresh perspectives : structures, functions, and biological properties of sulfated fucans and an overview of enzymes active toward this class of polysaccharide. Glycobiology (2003) 13 (6) : 29-40).

Les fucanes extraits des algues de l'ordre des Fucales (*Fucus sp., Ascophyllum sp.*) sont des polysaccharides hautement ramifiés (Berteau *et al* 2003; Patankar *et al.,* 1993; Bilan *et al.,* 2002, 2004, 2006) formés d'une chaîne principale composée de L-fucoses liés en α-(1,3) ou en β-(1,4) et variablement substitués par 1 ou 2 non sucres (sulfate ou acétate) sur le C2, le C3 ou le C4. Ce sont des homofucanes (Chevolot *et al.*, 1999, 2001; Bilan *et al.,* 2002, 2004, 2006; Chizhov *et al.,* 1999). Les ramifications peuvent être monosaccharidiques (Chizhov et al, A study of fucoidan from the brown seaweed Chorda filum. Carbohydrate Research (1999) 320 : 108-119)), trisaccharidique (Bilan et al, A highly regular fraction of a fucoidan from the brown seaweed Fucus distichus. Carbohydrate Research (2004), 339: 511-517) ou tétrasaccharidique (Bilan et al, Structure of a fucoidan from the brown seaweed Fucus evanescens.Ag. Carbohydrate Research (2002) 337: 719-730). Enfin, la présence de xylose ou galactose en faible quantité a pu être détectée mais leur localisation n'a pas été déterminée (Bilan *et al.,* 2002).

Comme autre polysaccharide d'origine naturelle, la composition selon l'invention peut également préférentiellement comprendre un polysaccharide sulfaté issu d'une microalgue, de préférence choisie parmi les microalgues rouges ou les cyanobactéries.
De manière tout aussi préférée, la composition selon l'invention comprend au moins un polysaccharide sulfaté issu
- d'une microalgue rouge choisie parmi les espèces appartenant au genre *Porphyridium* ou au genre *Rhodella* ;
- d'une cyanobactérie choisie parmi les espèces appartenant au genre *Arthospira* comme *Arthospira platensis.*

Les exopolysaccharides produits par des microalgues rouges marines sont principalement décrits chez les espèces appartenant aux genres *Porphyridium* et *Rhodella.* Ces polysaccharides sont des hétéropolymères variablement sulfatés et composés de monosaccharides neutres (xylose, glucose, galactose, mannose, arabinose, fucose), de monosaccharides méthylés (3-O-methyl xylose, 3-O et 4-O-methyl galactose), d'acides uroniques (acide glucuronique) et d'acides uroniques méthylés (2-O-methyl-acide glucuronique). Les polysaccharides des différentes espèces présentent plusieurs types de liaisons glycosidiques mais ont comme point commun la présence de blocs constitutifs constitués d'un disaccharide qualifié d'acide aldobiouronique (Arad et al, Red microalgal cell-wall polysaccharides: biotechnological aspects. Current opinion in Biotechnology (2010) 21: 358-364; Capek et al, The extracellular proteoglycan produced by Rhodella grisea. International Journal of Biological macromolecules (2008) 43, 390-393).

Les cyanobactéries produisent des exopolysaccharides de structures très complexes et caractérisées par la présence d'acides uroniques (acides glucuronique et galacturonique) et de groupements sulfate qui confèrent une nature anionique au polysaccharide. Ils sont en outre caractérisés par la présence de taux importants de groupements acétate, de fractions peptidiques et de desoxy-monosaccharides. La plupart des exopolysaccharides décrits chez les cyanobactéries sont composés d'au moins 6 monosaccharides différents et à ce jour 12 monosaccharides ont pu être identifiés chez les EPS de cyanobactéries. Parmi les hexoses on trouve le glucose, le galactose, le mannose et le fructose. Les pentoses sont quant à eux principalement représentés par le ribose, le xylose et l'arabinose alors que les desoxyoses identifiés sont le fucose, le rhamnose et le méthyl rhamnose. Dans certains cas la présence de monosaccharides modifiés comme la N-acétyl glucosamine, le 2,3-O-méthyl rhamnose et le 3-O-methyl glucose ont été décrits. Ces différents monosaccharides sont liés par une large gamme de liaisons glycosidiques (Pereira et al, Complexity of cyanobacterial exopolysaccharides : composition, structures, inducing factors and putative genes involved in their biosynthesis and assembly. FEMS Microbiol. Rev. (2009) 33 : 917-941).

De manière également avantageuse, la composition selon l'invention peut comprendre au moins un polysaccharide sulfaté obtenu par modification chimique d'un polysaccharide. Le polysaccharide est alors obtenu après avoir été modifié par voie chimique.

De manière habituelle, la composition selon l'invention comprend au moins un polysaccharide sulfaté dont le taux de sulfatation en poids est supérieur ou égal à 5%, de préférence ce taux va de 5 à 50%, avantageusement de 5 à 30%.

La composition selon l'invention peut comprendre des quantités relativement importantes d'au moins un polysaccharide sulfaté.
Généralement, la concentration massique de polysaccharide sulfaté dans la composition selon l'invention va de 50 à 1 000 µg/mL, préférentiellement de 100 à 1 000 µg/mL, avantageusement de 100 à 200 µg/mL.

La composition selon l'invention comprend également au moins un ingrédient alimentaire combiné à au moins un polysaccharide sulfaté.

De manière avantageuse, la concentration en poids de l'ingrédient alimentaire dans la composition selon l'invention va de 98% à 99,99%.

De manière générale, l'ingrédient alimentaire de la composition selon l'invention est un ingrédient qui peut être utilisé dans une composition alimentaire à destination de l'homme ou de l'animal. Cet ingrédient alimentaire répond notamment aux conditions de sécurité et de non-toxicité liées à une telle utilisation.
De manière préférée, la composition selon l'invention comprend au moins un ingrédient choisi parmi un agent protéique alimentaire, un sucre dont le saccharose, du miel ou un de leurs mélanges.

De manière avantageuse, la composition selon l'invention comprend un ingrédient alimentaire dont la concentration en poids dans la composition va de 98% à 99,99%, de préférence de 99 à 99,9%.

De manière préférée, la composition selon l'invention comprend un ingrédient alimentaire choisi parmi le miel, le saccharose, le fructose, le glucose ou le maltose, de préférence le saccharose, le miel ou leurs mélanges.

Les mélanges préférés d'ingrédient alimentaires pour la composition selon l'invention sont choisis parmi les mélanges de protéines, de miel et de saccharose ; les mélanges de miel et de saccharose. La composition selon l'invention peut comprendre d'autres combinaisons d'ingrédients alimentaires.

Dans la composition selon l'invention le ratio en poids (polysaccharide sulfaté/ingrédient alimentaire) peut varier de manière relativement importante. Généralement, le ratio en poids (polysaccharide sulfaté/ingrédient alimentaire) dans la composition selon l'invention va de 0,0001 à 0,002, préférentiellement de 0,0002 à 0,001, avantageusement de 0,0002 à 0,0005.

La forme de la composition peut être définie de manière assez large. Ainsi, la composition selon l'invention peut notamment se présenter sous forme solide, pâteuse ou liquide. La divulgation concerne également une méthode de préparation d'une composition selon l'invention.

De manière générale, la méthode de préparation selon l'invention comprend le mélange d'au moins un polysaccharide sulfaté et d'au moins un ingrédient alimentaire.

La méthode de préparation de la composition selon l'invention peut notamment comprendre une étape de sulfatation d'au moins un polysaccharide.

Cette étape de sulfatation peut être appliquée à un polysaccharide peu ou pas sulfaté ou à un polysaccharide sulfaté afin d'en accroître le taux de sulfatation.

De manière préférée, la méthode de préparation d'une composition selon l'invention comprenant un polysaccharide sulfaté issu d'une macroalgue, comprend:
- la mise en solution du polysaccharide sulfaté de la macroalgue, éventuellement dépigmentée préalablement, par chauffage à reflux dans l'eau à une température allant de 50 à 100 °C,
- la précipitation par au moins un solvant polaire ou par dialyse du polysaccharide sulfaté,
- le séchage du polysaccharide sulfaté.

La méthode de préparation selon l'invention peut comprendre une étape préalable de dépigmentation de la macroalgue, en particulier au moyen d'au moins un solvant qui peut être choisi parmi l'acétone ou le chloroforme.

De manière avantageuse, la précipitation peut être réalisée au moyen d'un solvant polaire, par exemple l'éthanol ou le propanol.

De manière également avantageuse, le séchage peut être réalisé par lyophilisation ou au moyen d'une étuve, notamment à une température de 50°C.

La méthode de préparation selon l'invention peut être renouvelée plusieurs fois afin d'obtenir un degré de pureté du polysaccharide satisfaisant.

De manière également préférée, la méthode de préparation d'une composition selon l'invention comprenant un polysaccharide sulfaté issu d'une microalgue, comprend:
- la culture de la microalgue, de préférence dans un photobioréacteur,
- la concentration du milieu de culture après extraction de la biomasse, sous vide et à une température allant de 30°C à 50°C,
- l'extraction du polysaccharide sulfaté par diafiltration, précipitation ou dialyse,
- le séchage du polysaccharide sulfaté.

De manière avantageuse, la concentration du milieu est réalisée à une température de 40°C.

De manière également avantageuse, l'extraction par précipitation peut être effectuée par centrifugation.

De manière également avantageuse, le séchage peut être réalisé par lyophilisation ou au moyen d'une étuve, notamment à une température de 50°C.

La méthode de préparation selon l'invention peut être renouvelée plusieurs fois afin d'obtenir un degré de pureté du polysaccharide satisfaisant.

De manière également préférée, la méthode de préparation d'une composition selon l'invention met en oeuvre un polysaccharide sulfaté obtenu par modification chimique d'un polysaccharide.

Ce polysaccharide sulfaté peut être préparé par un procédé comprenant une étape de sulfatation. Cette étape de sulfatation peut être effectuée par différentes méthodes connues en tant que telles. On peut notamment réaliser cette sulfatation au moyen d'un complexe SO₃/pyridine ou SO₃/DMF.

L'objectif de cette étape de sulfatation est de conférer au polysaccharide un taux de sulfatation suffisant pour l'utilisation de la composition selon l'invention ou encore cet objectif vise à augmenter le taux de sulfatation du polysaccharide.

L'invention concerne également une composition selon l'invention pour son utilisation :
- antiparasitaire chez l'homme ou l'animal ;
- dans le traitement ou la prévention d'une infection provoquée par au moins une microsporidie chez l'homme ou l'animal ;
- dans le traitement ou la prévention chez l'abeille d'une infection provoquée par la microsporidie *Nosema,* de préférence *Nosema ceranae* ou *Nosema apis ;*
- pour stimuler les défenses immunitaires de l'abeille.
La divulgation concerne également l'utilisation d'une composition selon l'invention ainsi qu'une composition selon l'invention pour son utilisation pour la production de miel, en présence d'une infection provoquée par au moins un parasite, et comprenant l'administration à des abeilles d'une composition selon l'invention. La divulgation concerne également l'utilisation d'une composition selon l'invention ainsi qu'une composition selon l'invention pour son utilisation pour maintenir la pollinisation des plantes par des abeilles infectées par au moins un parasite, et comprenant l'administration à des abeilles d'une composition selon l'invention.

L'invention concerne également une composition selon l'invention pour son utilisation antérieure, simultanée, complémentaire, séquencée, alternée ou postérieure à un traitement pour lutter contre *Varroa destructor.*

*Varroa destructor* est un acarien hématophage parasite des abeilles adultes, des larves et des nymphes.

Ainsi, l'utilisation d'une composition comprenant au moins un polysaccharide sulfaté et au moins un ingrédient alimentaire est susceptible de maintenir dans le temps, voire de favoriser l'efficacité d'un traitement pour lutter contre *Varroa destructor.*

La composition utilisée selon l'invention est définie selon l'ensemble des caractéristiques générales, avantageuses ou préférées de la composition selon l'invention.

La divulgation décrit également un kit comprenant :
- une composition selon l'invention,
- un agent pour la lutte contre ou la prévention du *Varroa destructor,* ainsi qu'un tel kit pour son utilisation dans la prévention ou le traitement des infections issues de la microsporidie *Nosema* et de *Varroa destructor* chez l'abeille.

L'invention concerne également un polysaccharide sulfaté défini pour la composition selon l'invention, pour son utilisation :
- antiparasitaire chez l'homme ou l'animal ;
- dans le traitement ou la prévention d'une infection provoquée par au moins une microsporidie chez l'homme ou l'animal ;
- dans le traitement ou la prévention chez l'abeille d'une infection provoquée par la microsporidie *Nosema,* de préférence *Nosema ceranae* ou *Nosema apis* ;
- pour stimuler les défenses immunitaires de l'abeille.

L'invention concerne également un polysaccharide sulfaté défini pour la composition selon l'invention pour son utilisation antérieure, simultanée, complémentaire, séquencée, alternée ou postérieure à un traitement pour lutter contre *Varroa destructor.*

Le polysaccharide selon l'invention est défini selon l'ensemble des caractéristiques générales, avantageuses ou préférées du polysaccharide de la composition selon l'invention.

Les exemples qui suivent sont donnés à titre d'illustration des différents aspects de l'invention.

### Préparation des polysaccharides sulfatés

### • Polysaccharide sulfaté 1 (PS1) : iota carraghénane

L'extraction a été basée sur une extraction alcaline (NaOH 0.1 à 1 M) à chaud (60 à 90°C) pendant plusieurs heures, d'un mélange d'algues rouges marines appartenant aux genres *Euchema, Kappaphycus, Chondrus* et *Betaphycus.* La suspension obtenue a été filtrée afin d'éliminer les débris cellulaires et précipitée par addition d'isopropanol. Le précipité a ensuite été pressé et séché.

Les iota carraghenanes comprennent une unité de répétition 3-β-D-galactopyranoses et 4-α-D-galactopyranoses alternant régulièrement. Cette dernière unité est sous forme anhydrogalactose à taux de sulfatation significatifs (30 à 50% en moyenne). Le taux moyen de sulfatation est de l'ordre de 30 %

### • Polysaccharide sulfaté 2 (PS2)

L'extraction a été réalisée après culture d'une microalgue rouge de type *Rhodella violacea* (souche LMGE) sur milieu Hemerick ou f/2.
L'extraction de la biomasse issue de cette culture a été réalisée par centrifugation à 10 000 g x 30 min à 10°C. Le surnageant a ensuite été concentré (x2) sous vide à 60°C puis dialysé 72 heures contre de l'eau milliQ (9 bains) puis le retentât a été séché par lyophilisation.

La composition en monosaccharides du PS2 ainsi obtenu a été déterminée par chromatographie ionique : xylose (20%), galactose (3%), glucose (0.5%), acide glucuronique (2.5%), arabinose (2%), rhamnose (3%). Les pourcentages sont des pourcentages massiques.

### • Polysaccharide sulfaté 3 (PS3)

L'extraction a été réalisée après culture sur milieu f/2 d'une microalgue rouge de type *Rhodella violacea* (kornmann) Wehrmeyer CCAP 1388/5. L'extraction de la biomasse issue de cette culture a été réalisée par centrifugation à 10 000 g x 30 min à 10°C. Le surnageant a ensuite été concentré (x2) sous vide à 60°C puis dialysé 72 heures contre de l'eau milliQ (9 bains) puis le retentât a été séché par lyophilisation.

La composition en monosaccharides du PS3 ainsi obtenu a été déterminée par chromatographie ionique : xylose (20%), galactose (3%), glucose (0.5%), acide glucuronique (2.5%), arabinose (2%), rhamnose (3%). Les pourcentages sont des pourcentages massiques.

### • Polysaccharide sulfaté 4 (PS4)

L'extraction a été réalisée après culture sur milieu f/2 d'une microalgue de type *Rhodella maculata* CCAP 1388/2 (cf Evans et al, Studies on the synthesis and composition of extracellular mucilage in the unicellular red alga Rhodella, J.Cell Sci, 1974, 16, 1-21).

L'extraction de la biomasse issue de cette culture a été réalisée par centrifugation à 10 000 g x 30 min à 10°C. Le surnageant a été concentré (x2) sous vide à 60°C puis dialysé 72 heures contre de l'eau milliQ (9 bains) puis le retentât a été séché par lyophilisation.

Les exopolysaccharides de *Rhodella maculata* comprennent des glucides (50%), des protéines (16%) et une part significative de sulfate (10%). Le monosaccharide majoritaire est le xylose. Des acides uroniques, du galactose et du glucose ont également été identifiés.

### • Polysaccharide sulfaté 5 (PS5)

L'extraction a été réalisée après culture sur milieu f/2 d'une microalgue de type *Porphyridium purpureum* (Bory) Drew & Ross (1965) CCAP 1380/1 A.

L'extraction de la biomasse issue de cette culture a été réalisée par centrifugation à 10 000 g x 30 min à 10°C. Le surnageant a ensuite été concentré (x2) sous vide à 60°C puis dialysé 72 heures contre de l'eau milliQ (9 bains) puis le retentât a été séché par lyophilisation.

### • Polysaccharide sulfaté 6 (PS6)

L'extraction a été réalisée après culture sur milieu f/2 d'une microalgue de type *Porphyridium marinum* Kylin (1937) CCAP 1380/10.

L'extraction de la biomasse issue de cette culture a été réalisée par centrifugation à 10 000 g x 30 min à 10°C. Le surnageant a été concentré (x2) sous vide à 60°C puis dialysé 72 heures contre de l'eau milliQ (9 bains) puis le retentât a été séché par lyophilisation.

### • Polysaccharide sulfaté 7 (PS7)

L'extraction a été réalisée après culture sur milieu Zarouk d'une cyanobactérie de type *Arthrospira platensis* PCC8005. L'extraction de la biomasse issue de cette culture a été réalisée par filtration sur verre fritté de porosité (40 - 100 µm). Le permeat a ensuite été concentré (x2) sous vide à 60°C puis dialysé 72 heures contre de l'eau milliQ (9 bains) puis le retentât a été séché par lyophilisation.

Les exopolysaccharides d'*Arthrospira platensis* sont généralement considérés comme des hétéropolysaccharides anioniques sulfatés. Les principaux monosaccharides identifiés sont le galactose (14.9%), le xylose (14.3%), le glucose (13.2%), le fructose (13.2%), le rhamnose (3.7%), l'arabinose (1%), le mannose (0.3%) et des acides uroniques (13.5% représentés pas l'acide galacturonique et glucuronique) (Trabelsi et al., Partial characterization of extracellular polysaccharide produced by Artrospira platensis. Biotechnol. Bioproc. Eng, 2009,14, 27-31).

### • Polysaccharide sulfaté 8 (PS8)

Des algues séchées de type *Halymenia durvillei* ont été lavées à l'eau puis séchées à l'étuve à 60°C.

L'extraction des polysaccharides a ensuite été réalisée par chauffage à reflux dans l'eau milliQ (ratio 1:20) pendant 4 heures à 90°C puis une filtration sur filtres de porosités décroissantes (de 160 à 40 µm) a été effectuée. Le filtrat ainsi obtenu a été centrifugé à 10 000 x g pendant 30 min (température ambiante). Le surnageant a ensuite été précipité par addition de 3 volumes d'éthanol à 96 %. Le précipité a ensuite collecté par pressage ou centrifugation (10 000 x g, 20 min, température ambiante) puis séché par lyophilisation.

Le PS8 comprend des unités 3-β-d-galactopyranose et des unités 4-α-D/L-galactopyranose.L
L'unité 3-β-D-galactopyranose est sulfatée en position 2 (26%) et 2/6 (58%).
L'unité 4-α-D/L-galactopyranose est sulfatée en 6 (19%) et en 2,6 (47%) et certains résidus sont de type 3,6 anhydrogalactopyranose.
Le polysaccharide est branché par des monosaccharides de type galactose, xylose, arabinose et fucose en quantités mineures et du pyruvate a également été détecté (1.8 %).

### • Polysaccharide sulfaté 9 (PS9)

L'extraction a été réalisée sur une algue sèche de type *Ulva lactuca* dépigmentée par traitement éthanol, acétone et chloroforme. L'extraction a été effectuée par incubation à reflux pendant 3 heures à 90°C dans une solution d'oxalate de sodium 50 mM, pH6. Après centrifugation à 12 000 x g, 30 min et 4°C, le surnageant a été précipité à l'isopropanol, collecté par centrifugation puis lyophilisé.

Le polysaccharide PS 9 est désigné par le terme ulvane et est constitué d'acide D-glucuronique, d'acide D-irduronique, de D-xylose, de L-rhamnose et de sulfate et est désigné comme un xylorhamnoglycuronane sulfaté. Des unités de répétition qualifiées d'acides aldobiouroniques ont pu être identifiées (Ray et al, Cell-wali polysaccharides from the marine green alga Ulva « rigida » (Ulvales chlorophytal) chemical structure of ulvan. Carbohydrate Research, 1995, 274: 313-318).

### Exemple 1 : évaluation de la cytotoxicité de polysaccharides sulfatés selon l'invention

### Culture de cellules HFF (Human Foreskin Fibroblast) en plaque 96 puits

A partir d'une boîte de cellules HFF de 75 cm² à confluence, le milieu de culture a été enlevé, puis a été ajouté 2 mL de trypsine-EDTA (trypsine 0,025% et EDTA 0,01%) (à 37°C). La boîte a ensuite été rincée brièvement et la trypsine a été enlevée. 2 mL de trypsine-EDTA ont ensuite été ajoutés et la boîte a été incubée 5 min dans l'étuve à 37°C.

5 mL de milieu de culture (MEM (PARA Laboratories GmbH) + Glutamine (2 mM) + Fungizone (2,5 µg/mL) + Penicilline (100 unités/mL) + Streptomycine (100 µg/mL) + Sérum de veau foetal décomplémenté 15 %) ont été ajoutés puis la boîte a été rincée à la pipette. Le milieu et les cellules ont été récupérés dans un tube. Un comptage sur cellule de Malassez a ensuite été réalisé.

Les cellules ont ensuite été centrifugées à 200 x g, pendant 7 min, puis le surnageant a été enlevé. Le culot a été repris dans le milieu de culture de façon à obtenir 10⁵ cellules/mL.

200 µL par puits de cette suspension ont été distribués en laissant vides les puits des colonnes 1 et 12 et des rangées A et H. Les puits laissés vides ont été remplis avec 200 µL de milieu de culture afin d'éviter l'évaporation.

### Ajout des polysaccharides sulfatés

Lorsque les cellules ont été à confluence dans les puits (environ 48h), le milieu a été aspiré avec une trompe à vide puis 200 µL de milieu de culture contenant les divers polysaccharides à tester (ceux-ci sont testés à 50, 100, et 200 µg/mL) ont été ajoutés. Chaque concentration a été testée en triplicat. Chaque plaque devait contenir un témoin négatif (milieu de culture sans extrait), un témoin positif (milieu de culture contenant 20% de DMSO), et un témoin fumagilline (à 1 µg/mL). Les résultats sont rassemblés dans le tableau 1.

**Tableau 1**

| | Viabilité (%) | Conclusion |
|---|---|---|
| Témoin cellules saines | 100 +/- 2,3 | Témoin validé |
| Témoin positif DMSO | 6,8 +/- 1,4 | Cytotoxique : témoin validé |
| Témoin fumagilline | 99,1 +/- 12,2 | Non-cytotoxique : témoin validé |

### Test de cytotoxicité

Après 96 h d'incubation, le milieu contenu dans les puits a été aspiré avec une trompe à vide puis 200 µL de milieu de culture frais et 50 µL de TCA (acide trichloracétique) à 50% ont été ajoutés et le mélange a été laissé 5 min à température ambiante puis 2 h à 4°C.

Le surnageant a été ôté avec une trompe à vide et lavé 5 fois (par retournement de la plaque 96 puits) avec 100 µL d'eau distillée puis la plaque a été séchée (1 h dans la trompe à vide, ou 2 h retournée sur la paillasse). La plaque une fois séchée a pu ainsi être conservée à température ambiante jusqu'à l'utilisation.

100 µL de sulforhodamine B (0,4% dans de l'acide acétique 1%) ont été ajoutés et le mélange a été incubé 20 min à température ambiante. La sulforhodamine B a été retirée à l'aide d'une trompe à vide. Un lavage répété 5 fois (par retournement de la plaque 96 puits) avec 100 µL d'acide acétique 1% a été effectué puis la plaque a été séchée 5 min à température ambiante en la retournant.

Les protéines ont été solubilisées à l'aide de 200 µL de Tris-Base (10 mM) pendant 5 min sur un plateau agitant à température ambiante. La DO (Densité Optique) à 550 nm a été mesurée à l'aide d'un lecteur de microplaques (Multiskan FC357, Thermo Scientific).

Les résultats sont présentés dans le tableau 2.

La moyenne de la DO et l'écart type ont été calculés pour chaque échantillon testé afin de conclure sur la toxicité des produits.

**Tableau 2**

| | Viabilité (%) à 50 µg/mL | Viabilité (%) à 100 µg/mL | Viabilité (%) à 200 µg/mL | Conclusion |
|---|---|---|---|---|
| PS1 | 119,1 +/- 1,8 | 156 +/- 8 | 110+/-5 | Non-cytotoxique |
| PS2 | 95,6 +/- 13,7 | 89,2 +/- 6 | 69,2 +/- 7,8 | Non-cytotoxique jusqu'à 100 µg/mL |
| PS3 | 101,3 +/- 8,5 | 108,1 +/- 20,8 | 110 +/- 13,5 | Non-cytotoxique |
| PS4 | 94,2 +/- 5,2 | 98,7 +/- 20,8 | 97,3 +/- 8,8 | Non-cytotoxique |
| PS5 | 81,1 +/-2,9 | 74,3 +/- 10 | 87,5 +/- 15,6 | Considéré comme non-cytotoxique |
| PS6 | 84,4 +/- 13 | 89 +/- 6,4 | 63,7 +/- 5 | Non-cytotoxique jusqu'à 100 µg/mL |
| PS7 | 144,7 +/- 30,6 | 136,7 +/- 21,8 | 139,6 +/- 18,2 | Non-cytotoxique |
| PS8 | 143,7 +/- 35,7 | 142,2 +/- 31 | 96,7 +/- 22,3 | Non-cytotoxique |
| PS9 | 149,4 +/- 16,1 | 141,4 +/- 18,3 | 124,5 +/- 20,2 | Non-cytotoxique |

Les résultats montrent que les polysaccharides sulfatés selon l'invention ne présentent pas de cytotoxicité, notamment jusqu'à 100 µg/mL.

### Exemple 2: évaluation de l'activité anti-parasitaire in vitro sur la microsporidie Encephalitozoon cuniculi, de polysaccharides sulfatés selon l'invention

### Culture de cellules HFF (Human Foreskin Fibroblast) en plaques 48 puits

A partir d'une boîte de cellules HFF de 75 cm² à confluence, le milieu de culture a été enlevé puis 2 mL de trypsine-EDTA (trypsine 0,025% et EDTA 0,01%) (à 37°C) ont été ajoutés.

La boîte a ensuite été brièvement rincée puis la trypsine a été retirée. 2 mL de trypsine-EDTA (trypsine 0,025% et EDTA 0,01%) ont ensuite été ajoutés et la boîte a été incubée 5 min dans l'étuve à 37°C. 5 mL de milieu de culture (MEM (PARA Laboratories GmbH) + Glutamine (2 mM) + Fungizone (2,5 µg/mL) + Penicilline (100 unités/mL) + Streptomycine (100 µg/mL) + Sérum de veau foetal décompléménté 15 %) ont été ajoutés et la boîte a été rincée à la pipette. Le milieu et les cellules ont été récupérés dans un tube. Un comptage sur cellule de Malassez a été effectué. Puis les cellules ont été centrifugées à 200 x g pendant 7 min. Le surnageant a ensuite été retiré et le culot a été repris dans le milieu de culture de façon à obtenir 10⁵ cellules/mL.

400 µL de cette suspension/puits a été distribué en laissant vides les puits des colonnes 1 et 8 et des rangées A et F. Les puits laissés vides ont été remplis avec 400 µL de milieu de culture afin d'éviter l'évaporation.

### Test ELISA sur microsporidies

Lorsque les cellules ont été à confluence dans les puits (environ 48h), le milieu a été aspiré à l'aide d'une trompe à vide. Puis 400 µL de milieu de culture contenant les divers polysaccharides à tester (ceux-ci ont été testés à la plus forte concentration non-cytotoxique, c'est-à-dire 100 ou 200 µg/mL) ont été ajoutés, puis incubés 2 h à l'étuve à 37°C.

Parallèlement, une solution de spores *d'Encephalitozoon cuniculi* (obtenues à partir de surnageant de culture entretenues au laboratoire) à une concentration de 5.10⁵ spores /mL a été pré-incubée 2 h à 37°C dans du milieu de culture contenant les polysaccharides à tester. Des spores ont également été pré-incubées dans les mêmes conditions mais sans polysaccharide, ces spores ayant servi à infecter des cellules utilisées pour réaliser le témoin négatif cité plus bas. Celle-ci a ensuite été mise en contact pendant 1 h à 37°C avec les cellules, le polysaccharide étant toujours présent.

Après un lavage au PBS afin d'éliminer les spores non adhérentes, les divers polysaccharides ont été rajoutés et laissés ainsi le temps de la manipulation (5 jours). Chaque polysaccharide a été testé en triplicat. Chaque plaque contenait un témoin « cellules saines » non infectées, un témoin négatif (cellules infectées sans traitement) et un témoin positif (cellules infectées incubées en présence de fumagilline à 1 µg/mL).

A la fin de la manipulation, le milieu de culture de chaque puits a été éliminé et un lavage avec 200 µL de PBS a été effectué. Les puits ont été fixés individuellement par 100 µL de méthanol durant 20 minutes, à -80 °C puis les cellules ont été saturées par 100 µL d'une solution de saturation (Tris 100 mM, BSA 2%) pendant une nuit à 4°C.

La solution de saturation a ensuite été éliminée à l'aide d'une trompe à vide, puis 100 µL de sérum de lapin infecté naturellement (anticorps primaires) dilué au 1/1000^{ème} dans le tampon de dilution pour anticorps (Tris 10 mM pH=7,4, NaCl 150 mM, Tween 20 0,05 %, BSA 0,2 %) ont été ajoutés. Le mélange a été incubé pendant 2 h à 37°C. Puis un lavage répété 5 fois avec 200 µL de la solution de lavage (Tween 20 0,05%, Tris 10 mM pH=9,8) a été effectué. 100 µL de l'anticorps secondaire anti-IgG de lapin (IgG AP-conjugate Promega 1mg/mL) couplé à la phosphatase alcaline dilué au 1/10 000^{ème} dans le tampon de dilution pour anticorps ont été ajoutés. Le mélange a été incubé pendant 1 h, à 37°C puis un lavage répété 5 fois avec 200 µL de la solution de lavage a été effectué.

200 µL de MUP à 10 mM (4-méthylumbelliferyl phosphate) dilué au 1/100^{ème} dans la solution de révélation (MgCl₂ 1 mM, Na₂CO₃ 50 mM pH=9,8) ont été ajoutés, puis le mélange a été incubé pendant 30 min à l'obscurité, sous agitation légère.

La lecture a été réalisée au fluoroscan avec une longueur d'onde d'excitation et d'émission de 355 nm et 460 nm, respectivement.

Les résultats sont présentés dans le tableau 3.

Les données d'inhibition de développement parasitaire ont été calculées en tenant compte des informations suivantes : le témoin « cellules saines » correspond au blanc, le témoin négatif (cellules infectées sans traitement) correspond à 100% de développement parasitaire.

La capacité d'inhibition des polysaccharides testés a été évaluée selon l'échelle suivante :
- < 30 % : faible inhibition,
- entre 30 et 40 % : inhibition moyenne,
- entre 40 et 50 % : inhibition satisfaisante,
- entre 50 et 70 % : forte inhibition,
- entre 70 et 90 % : très forte inhibition,
- > 90% : excellente inhibition.

**Tableau 3**

| Produit | Inhibition du développement parasitaire (%) | Conclusion |
|---|---|---|
| PS1 (200 µg/mL) | 34,5 +/- 5 | inhibition moyenne |
| PS2 (100 µg/mL) | 34,1 +/- 11,9 | inhibition moyenne |
| PS3 (200 µg/mL) | 46,8 +/- 8,7 | inhibition satisfaisante |
| PS4 (200 µg/mL) | 29,9 +/- 4 | inhibition moyenne |
| PS5 (200 µg/mL) | 99,4 +/- 1,1 | excellente inhibition |
| PS6 (100 µg/mL) | 90,3 +/- 16,7 | excellente inhibition |
| PS7 (200 µg/mL) | 48,3 +/- 11,3 | inhibition satisfaisante |
| PS8 (200 µg/mL) | 35,7 +/- 7,4 | inhibition moyenne |
| PS9 (200 µg/mL) | 35,9 +/- 0,7 | inhibition moyenne |

Les résultats montrent que les polysaccharides sulfatés selon l'invention présentent une activité antiparasitaire sur microsporidie satisfaisante. Les polysaccharides sulfatés issu d'une microalgue du genre *Porphyridium* montrent une excellente activité inhibitrice.

### Exemple 3: évaluation de l'activité inhibitrice in vivo de polysaccharides sulfatés selon l'invention sur la nosémose chez les abeilles

Les résultats présentés ici ont été obtenus sur des abeilles d'été.

Récupération d'abeilles émergentes (naissantes) et maintien en cagette d'élevage

Après avoir récupéré un cadre (ou plusieurs selon le besoin en abeilles) de couvain au rucher par l'intermédiaire d'un porte-cadre vitré, celui-ci a été placé à l'étuve à 34°C pendant une durée de 24 à 48 h de manière à laisser les abeilles émerger de leurs alvéoles. Puis les jeunes abeilles ont été récupérées sur le cadre à l'aide d'une pince d'entomologiste et placées dans les cagettes d'élevage à raison de 50 abeilles/cagette. 1/6^{ème} d'un bâtonnet de PseudoQueen (Contech enterprises, Canada) a été ajouté dans chacune des cagettes.

### Nourrissage des abeilles

Chacun des traitements polysaccharidiques a été réalisé en triplicat (3 cagettes/traitement). Chaque manipulation contenait un témoin « abeilles saines » (abeilles non infectées et non traitées), un témoin « abeilles infectées » (abeilles infectées expérimentalement et non traitées) et un témoin fumagilline ; ces témoins étant également en triplicat.

Les abeilles ont été nourries pendant 3 jours avant infection avec du sirop seul (témoins « abeilles saines » et « abeilles infectées »), avec du sirop additionné de 1 µg/mL de fumagilline (témoin fumagilline) ou avec du sirop contenant les divers polysaccharides sulfatés à tester (à la concentration non-cytotoxique la plus élevée, c'est-à-dire 100 ou 200 µg/mL). Le sirop était constitué de 50 % de saccharose, additionné de 1 % de protéines (Provita'bee, Laboratoires Corylis, France).

Le nourrissage des abeilles a été réalisé grâce à des tubes plastiques transparents de 5 mL percés à leur extrémité afin que les abeilles puissent se nourrir.

### Infection individuelle des abeilles par Nosema ceranae

Les abeilles ont jeûné pendant 30 à 60 min avant infection. Pendant ce temps, une solution de spores de *Nosema ceranae* a été préparée en diluant celles-ci dans du sirop de nourrissage à raison de 125 000 spores pour 3 µL. Chaque abeille a été infectée par 125 000 spores.

Les abeilles ont ensuite été gazées au CO₂ (environ 1 min/cagette). Lorsque les abeilles ont commencé à se réveiller, celles-ci ont été prélevées à l'aide d'une pince d'entomologiste. Les abeilles ont ensuite été infectées individuellement par voie orale, à l'aide d'une pipette contenant 3 µL de la solution de spores préparée (en veillant à ce que la totalité des 3 µL soient bue par l'abeille). La même manipulation a été effectuée avec les cagettes du témoin « abeilles saines » en veillant à ne donner que du sirop de nourrissage seul. 45 abeilles ont été placées par cagette.

Après l'étape d'infection, à chacune des cagettes ont été ajoutés les tubes de nourrissage correspondant aux différentes conditions (sirop seul pour témoins « abeilles saines » et « abeilles infectées », sirop additionné de 1 µg/mL de fumagilline pour témoin fumagilline, sirop contenant les divers polysaccharides sulfatés). Les abeilles ont été mises à l'étuve à 33°C (hygrométrie 60 - 70 %), et nourries en continu pendant une durée de 20 jours.

### Suivi de la manipulation durant la phase de post-infection

Au cours de la phase de post-infection (d'une durée de 20 jours), les abeilles mortes ont été comptées et enlevées quotidiennement dans chacune des cagettes afin d'effectuer le suivi de mortalité. Les tubes de nourrissage ont été remplacés tous les deux jours jusqu'à la fin de la manipulation. Il était possible de peser les tubes de nourrissage dans l'optique d'un suivi de la consommation des différents traitements par les abeilles.

Au terme des 20 jours, 30 abeilles vivantes (10 abeilles/cagette) ont été sacrifiées pour chacune des conditions testées, afin de réaliser un comptage de spores de *Nosema ceranae* dans le but d'évaluer la charge parasitaire des abeilles. Pour cela l'intestin et l'ampoule rectale de chacune des abeilles ont été disséqués puis broyés au broyeur de Potter dans 100 µL de PBS. Après 3 lavages, le comptage a été réalisé sur cellules de Kovacs.

Les résultats sont présentés dans le tableau 4.

**Tableau 4**

| | Mortalité à 20 j post-infection (%) | Variation charge parasitaire |
|---|---|---|
| Témoin « abeilles infectées » | 53,3 +/- 13,6 | Aucune |
| Témoin fumagilline | 25,9 +/- 16,7 | Baisse de 63,4 % |
| PS1 (200 µg/mL) | 60 +/- 8 | Baisse de 26,4 % |
| PS3 (200 µg/mL) | 41,5 +/- 19 | Baisse de 0,6 % |
| PS5 (200 µg/mL) | 43,7 +/- 6,8 | Baisse de 20,4 % |
| PS6 (100 µg/mL) | 35,6 +/-10,2 | Baisse de 33,8 % |
| Mélange PS1-PS5 (100 µg/mL chacun) | 54,8 +/- 26,5 | Baisse de 6,25 % |

Les résultats montrent une baisse significative de la mortalité des abeilles lorsqu'elles ont été nourries avec une composition comprenant un polysaccharide sulfaté selon l'invention.

Le polysaccharide sulfaté PS6 issu de *Porphyridium marinum* montre notamment une baisse importante de la mortalité chez les abeilles.

Ainsi les polysaccharides sulfatés selon l'invention permettent de limiter le développement du parasite (illustré par la baisse du nombre de spores) et ainsi de diminuer significativement la mortalité chez les abeilles atteintes de nosémose.

## Revendications

1. Composition comprenant au moins un polysaccharide sulfaté et au moins un ingrédient alimentaire pour son utilisation dans le traitement ou la prévention d'une infection provoquée par au moins une microsporidie chez l'homme ou l'animal.

2. Composition pour une utilisation selon la revendication précédente dans laquelle l'ingrédient alimentaire est choisi parmi un agent protéique alimentaire, un sucre dont le saccharose, le miel ou leurs mélanges.

3. Composition pour une utilisation selon les revendications précédentes dans laquelle le polysaccharide sulfaté est d'origine naturelle.

4. Composition pour une utilisation selon les revendications 1 à 3 comprenant un polysaccharide sulfaté issu d'une macroalgue, de préférence choisie parmi les macroalgues rouges, les macroalgues vertes ou les macroalgues brunes ou issu d'une microalgue, de préférence choisie parmi les microalgues rouges ou les cyanobactéries ; ou encore un mélange de tels polysaccharides sulfatés.

5. Composition pour une utilisation selon la revendication précédente dans laquelle :
- la macroalgue rouge est choisie parmi les Rhodophycées productrices d'agar, de carraghénanes, de porphyranes, de funoranes ou de galactanes sulfatés complexes, préférentiellement du genre *Gracilaria, Halymenia, Gelidium, Pterocladia, Acanthopeltis, Campylaephora, Ceranium, Euchema , Chondrus, Porphyra, Laurencia, Furcellaria, Gloiopeltis* et *Iridea* ;
- la macroalgue verte est choisie parmi les Chlorophycées produisant des polysaccharides de type ulvanes, de préférence du genre *Ulva ;*
- la macroalgue brune est choisie parmi les Phéophycées productrices de fucanes sulfatés, préférentiellement du genre *Fucus, Aschophyllum* ou *Cladosiphon.*

6. Composition pour une utilisation selon la revendication 4 ou 5 dans laquelle le polysaccharide sulfaté issu d'une macroalgue est obtenu ou susceptible d'être obtenu par un procédé comprenant les étapes de :
- mise en solution du polysaccharide sulfaté de la macroalgue, éventuellement dépigmentée préalablement, par chauffage à reflux dans l'eau à une température allant de 50 à 100 °C,
- précipitation du polysaccharide sulfaté au moyen d'au moins un solvant polaire ou par dialyse,
- séchage.

7. Composition pour une utilisation selon la revendication 4 dans laquelle :
- la microalgue rouge est choisie parmi les espèces appartenant au genre *Porphyridium* ou au genre *Rhodella ;*
- la cyanobactérie est *Arthospira platensis.*

8. Composition pour une utilisation selon la revendication 4 ou 7 dans laquelle le polysaccharide sulfaté issu de la microalgue est obtenu ou susceptible d'être obtenu par une méthode comprenant
- la culture de la microalgue, de préférence dans un photobioréacteur,
- la concentration, après extraction de la biomasse, du milieu de culture sous vide et à une température allant de 30°C à 50°C,
- l'extraction du polysaccharide sulfaté par diafiltration, par précipitation ou par dialyse,
- le séchage du polysaccharide sulfaté.

9. Composition pour une utilisation selon les revendications 1 ou 8 dans laquelle
- le polysaccharide sulfaté est obtenu par modification chimique d'un polysaccharide ; ou
- le polysaccharide sulfaté est préparé ou susceptible d'être préparé par un procédé comprenant une étape de sulfatation ; ou
- le polysaccharide sulfaté a un taux de sulfatation en poids supérieur ou égal à 5%, de préférence allant de 5 à 50%, avantageusement de 5 à 30% ; ou
- la concentration massique de polysaccharide sulfaté dans la composition va de 50 à 2 000 µg/mL, préférentiellement de 100 à 1 000 µg/mL, avantageusement de 100 à 200 µg/mL ; ou
- le ratio en poids (polysaccharide sulfaté/ingrédient alimentaire) va de 0,0001 à 0,002, préférentiellement de 0,0002 à 0,001, avantageusement de 0,0002 à 0,0005 ; ou
- l'ingrédient alimentaire est choisi parmi le saccharose, le fructose, le glucose ou le maltose, de préférence le saccharose, le miel ou leurs mélanges ; ou
- la concentration en poids de polysaccharide sulfaté dans la composition va de 0,01 à 2%, préférentiellement de 0,1 à 1% ; ou
- la concentration en poids de l'ingrédient alimentaire dans la composition va de 98% à 99,99%.

10. Composition pour une utilisation selon l'une quelconque des revendications précédentes
- pour son utilisation dans le traitement ou la prévention chez l'abeille d'une infection provoquée par la microsporidie *Nosema,* de préférence *Nosema ceranae* ou *Nosema apis* ; ou
- pour son utilisation simultanée pour stimuler les défenses immunitaires de l'abeille ; ou
- pour son utilisation antérieure, simultanée, complémentaire, séquencée, alternée ou postérieure à un traitement pour lutter contre *Varroa destructor.*

11. Polysaccharide sulfaté défini par l'une quelconque des revendications 3 à 9 pour son utilisation dans le traitement ou la prévention d'une infection provoquée par au moins une microsporidie chez l'homme ou l'animal.

12. Polysaccharide sulfaté pour une utilisation selon la revendication 11
- pour son utilisation dans le traitement ou la prévention chez l'abeille d'une infection provoquée par la microsporidie *Nosema,* de préférence *Nosema ceranae* ou *Nosema apis* ; ou
- pour son utilisation simultanée pour stimuler les défenses immunitaires de l'abeille ; ou
- pour son utilisation antérieure, simultanée, complémentaire, séquencée, alternée ou postérieure à un traitement pour lutter contre *Varroa destructor.*

## Patentansprüche

1. Zusammensetzung, die wenigstens ein sulfatiertes Polysaccharid und wenigstens eine Nahrungsmittelzutat umfasst, zu ihrer Verwendung bei der Behandlung oder der Prävention einer durch wenigstens eine Mikrosporidie hervorgerufenen Infektion bei einem Menschen oder einem Tier.

2. Zusammensetzung zur Verwendung nach dem vorhergehenden Anspruch, bei der die Nahrungsmittelzutat aus einem proteinhaltigen Nahrungsmittel, einem Zucker einschließlich Saccharose, Honig oder deren Gemischen ausgewählt ist.

3. Zusammensetzung zur Verwendung nach den vorhergehenden Ansprüchen, bei der das sulfatierte Polysaccharid natürlichen Ursprungs ist.

4. Zusammensetzung zur Verwendung nach den Ansprüchen 1 bis 3, die ein sulfatiertes Polysaccharid, das aus einer Makroalge stammt, die vorzugsweise aus den roten Makroalgen, den grünen Makroalgen oder den braunen Makroalgen ausgewählt ist, oder das aus einer Mikroalge stammt, die vorzugsweise aus den roten Mikroalgen oder den Cyanobakterien ausgewählt ist; oder auch ein Gemisch aus solchen sulfatierten Polysacchariden umfasst.

5. Zusammensetzung zur Verwendung nach dem vorhergehenden Anspruch, bei der:
- die rote Makroalge aus den Rhodophyceae, die Agar, Carrageenane, Porphyrane, Funorane oder komplexe sulfatierte Galactane produzieren, vorzugsweise aus der Gattung *Gracilaria, Halymenia, Gelidium, Pterocladia, Acanthopeltis, Campylaephora, Ceranium, Euchema, Chondrus, Porphyra, Laurencia, Furcellaria, Gloiopeltis* und *Iridea* ausgewählt ist;
- die grüne Makroalge aus den Chlorophyceae, die Polysaccharide vom Typ der Ulvane produzieren, vorzugsweise aus der Gattung *Ulva* ausgewählt ist;
- die braune Makroalge aus den Phaeophyceae, die sulfatierte Fucane produzieren, vorzugsweise aus der Gattung *Fucus, Aschophyllum* oder *Cladosiphon* ausgewählt ist.

6. Zusammensetzung zur Verwendung nach Anspruch 4 oder 5, bei der das aus einer Makroalge stammende sulfatierte Polysaccharid durch ein Verfahren gewonnen wird oder gewonnen werden kann, das die folgenden Schritte umfasst:
- Inlösungbringen des sulfatierten Polysaccharids der gegebenenfalls zuvor depigmentierten Makroalge durch Erhitzen unter Rückfluss in Wasser bei einer Temperature von 50 bis 100°C,
- Präzipitation des sulfatierten Polysaccharids mithilfe wenigstens eines polaren Lösungsmittels oder durch Dialyse,
- Trocknung.

7. Zusammensetzung zur Verwendung nach Anspruch 4 bei der:
- die rote Mikroalge aus den Arten, die zur Gattung *Porphyridium* oder zur Gattung *Rhodella* gehören, ausgewählt ist;
- das Cyanobakterium *Arthospira platensis* ist.

8. Zusammensetzung zur Verwendung nach Anspruch 4 oder 7, bei der das aus der Mikroalge stammende sulfatierte Polysaccharid durch ein Verfahren gewonnen wird oder gewonnen werden kann, das umfasst:
- die Kultivierung der Mikroalge, vorzugsweise in einem Photobioreaktor,
- das Aufkonzentrieren, nach Extraktion der Biomasse, des Kulturmediums unter Vakuum und bei einer Temperatur von 30°C bis 50°C,
- die Extraktion des sulfatierten Polysaccharids durch Diafiltration, durch Präzipitation oder durch Dialyse,
- die Trocknung des sulfatierten Polysaccharids.

9. Zusammensetzung zur Verwendung nach den Ansprüchen 1 oder 8, bei der
- das sulfatierte Polysaccharid durch chemische Modifikation eines Polysaccharids gewonnen wird; oder
- das sulfatierte Polysaccharid durch ein Verfahren, das einen Schritt der Sulfatierung umfasst, hergestellt wird oder hergestellt werden kann; oder
- das sulfatierte Polysaccharid einen Sulfatierungsgrad von wenigstens 5 Gew.%, vorzugsweise von 5 bis 50 Gew.%, vorteilhafterweise von 5 bis 30 Gew.% aufweist; oder
- die Massenkonzentration des sulfatierten Polysaccharids in der Zusammensetzung 50 bis 2 000 µg/ml, vorzugsweise 100 bis 1 000 µg/ml, vorteilhafterweise 100 bis 200 µg/ml beträgt; oder
- das Gewichtsverhältnis (sulfatiertes Polysaccharid/Nahrungsmittelzutat) 0,0001 bis 0,002, vorzugsweise 0,0002 bis 0,001, vorteilhafterweise 0,0002 bis 0,0005 beträgt; oder
- die Nahrungsmittelzutat aus Saccharose, Fructose, Glucose oder Maltose, vorzugsweise Saccharose, Honig oder deren Gemischen ausgewählt ist; oder
- die gewichtsbezogene Konzentration des sulfatierten Polysaccharids in der Zusammensetzung 0,01 bis 2 Gew.%, vorzugsweise 0,1 bis 1 Gew.% beträgt; oder
- die gewichtsbezogene Konzentration der Nahrungsmittelzutat in der Zusammensetzung 98 Gew.% bis 99,99 Gew.% beträgt.

10. Zusammensetzung zur Verwendung nach einem der vorhergehenden Ansprüche
- zu ihrer Verwendung bei einer Biene bei der Behandlung oder der Prävention einer Infektion, die durch die Mikrosporidie *Nosema,* vorzugsweise *Nosema ceranae* oder *Nosema apis,* hervorgerufen wird; oder
- zu ihrer gleichzeitigen Verwendung zur Stimulation der Immunabwehr der Biene; oder
- zu ihrer Verwendung vor, gleichzeitig mit, ergänzend zu, sequentiell mit, alternierend mit oder nach einer Behandlung zur Bekämpfung von *Varroa destructor.*

11. Sulfatiertes Polysaccharid, das durch einen der Ansprüche 3 bis 9 definiert ist, zu seiner Verwendung bei der Behandlung oder der Prävention einer durch wenigstens eine Mikrosporidie hervorgerufenen Infektion bei einem Menschen oder einem Tier.

12. Sulfatiertes Polysaccharid zur Verwendung nach Anspruch 11
- zu seiner Verwendung bei einer Biene bei der Behandlung oder der Prävention einer Infektion, die durch die Mikrosporidie *Nosema,* vorzugsweise *Nosema ceranae* oder *Nosema apis,* hervorgerufen wird; oder
- zu seiner gleichzeitigen Verwendung zur Stimulation der Immunabwehr der Biene; oder
- zu seiner Verwendung vor, gleichzeitig mit, ergänzend zu, sequentiell mit, alternierend mit oder nach einer Behandlung zur Bekämpfung von *Varroa destructor.*

## Claims

1. A composition comprising at least one sulphated polysaccharide and at least one food ingredient, for use thereof in the treatment or prevention of an infection caused by at least one microsporidian pathogen in humans or animals.

2. A composition for use thereof according to the preceding claim, wherein the food ingredient is selected from a dietary protein agent, a sugar including saccharose (or sucrose), honey or the mixtures thereof.

3. A composition for use thereof according to the preceding claims, wherein the sulphated polysaccharide is of natural origin.

4. A composition for use thereof according to claims 1 to 3, comprising a sulphated polysaccharide derived from a macroalga, preferably selected from among the red macroalgae, the green macroalgae, or the brown macroalgae; or derived from a microalga, preferably selected from the red microalgae and cyanobacteria; or a mixture of such sulphated polysaccharides.

5. A composition for use thereof according to the preceding claim wherein:
- the red macroalga is selected from among the Rhodophyceae that produce agar, carrageenans, porphyrans, funorans, or complex sulphated galactans, preferably of the genera *Gracilaria, Halymenia, Gelidium, Pterocladia, Acanthopeltis, Campylaephora, Ceranium, Eucheuma, Chondrus, Porphyra, Laurencia, Furcellaria, Gloiopeltis* and *Iridea;*
- the green macroalga is selected from the Chlorophyceae that produce polysaccharides like ulvan, preferably of the genus *Ulva*;
- the brown macroalga is selected from the Pheophyceae that produce sulphated fucans, preferably of the genera *Fucus, Aschophyllum,* or *Cladosiphon.*

6. A composition for use thereof according to claim 4 or 5, wherein the sulphated polysaccharide derived from a macroalga is obtained or may possibly be obtained by a method including the following steps of:
- the preparation of solution of the sulphated polysaccharide from the macroalgae, possibly previously depigmented, by heating under reflux in water at a temperature ranging from 50°C to 100°C;
- the precipitation of the sulphated polysaccharide by at least one polar solvent or by means of dialysis;
- the drying of the sulphated polysaccharide.

7. A composition for use thereof according to claim 4, wherein:
- the red microalga is selected from the species belonging to the genus *Porphyridium* or the genus *Rhodella*;
- the cyanobacterium is *Arthospira platensis.*

8. A composition for use thereof according to claim 4 or 7, wherein the sulphated polysaccharide derived from the microalga is obtained or may possibly be obtained by a method including:
- the culturing of the microalga, preferably in a photobioreactor;
- the concentration of the culture medium, after extraction of the biomass, in vacuo and at a temperature ranging from 30°C to 50°C;
- the extraction of the sulphated polysaccharide by diafiltration, precipitation or dialysis;
- the drying of the sulphated polysaccharide.

9. A composition for use thereof according to claims 1 or 8, wherein
- the sulphated polysaccharide is obtained by chemical modification of a polysaccharide; or
- the sulphated polysaccharide is prepared or may possibly be prepared by a method including a step of sulphation; or
- the sulphated polysaccharide has a rate of sulphation on a weight basis that is greater than or equal to 5%, preferably ranging from 5% to 50%, advantageously from 5% to 30%; or
- the mass concentration of sulphated polysaccharide in the composition ranges from 50 to 2000 mg/mL, preferably from 100 to 1000 µg/mL, advantageously from 100 to 200 µg/mL;
- the (sulphated polysaccharide/food ingredient) ratio by weight ranges from 0.0001 to 0.002, preferably from 0.0002 to 0.001, advantageously from 0.0002 to 0.0005; or
- the food ingredient is selected from among saccharose, fructose, glucose, or maltose; preferably saccharose, honey or mixtures thereof; or
- the concentration by weight of sulphated polysaccharide in the composition ranges from 0.01% to 2%, preferably from 0.1% to 1%; or
- the concentration by weight of the food ingredient in the composition ranges from 98% to 99.99%.

10. A composition for use thereof according to any one of the preceding claims:
- for use thereof in the treatment or prevention of an infection caused in honey bees by the microsporidium *Nosema,* preferably *Nosema ceranae* or *Nosema apis*; or
- for the simultaneous use thereof for stimulating the immune defence system of the honey bee; or
- for use thereof, on a prior -, simultaneous -, supplementary -, sequenced -, alternating -, or subsequent basis, with respect to a treatment for fighting against *Varroa destructor.*

11. A sulphated polysaccharide as defined by any one of claims 3 to 9 for use thereof in the treatment or prevention of an infection caused by at least one microsporidium in humans or animals.

12. A sulphated polysaccharide for use thereof according to claim 11 :
- for use thereof in the treatment or prevention of an infection caused in honey bees by the microsporidium *Nosema,* preferably *Nosema ceranae* or *Nosema apis*; or
- for the simultaneous use thereof for stimulating the immune defence system of the honey bee; or
- for use thereof, on a prior -, simultaneous -, supplementary -, sequenced -, alternating -, or subsequent basis, with respect to a treatment for fighting against *Varroa destructor.*
